# EUROPEAN PATENT SPECIFICATION

(11) **EP 3 661 428 B1**
(45) Date of publication and mention of the grant of the patent: **21.07.2021**
(21) Application number: 18797491.0
(22) Date of filing: 22.10.2018
(51) Int. Cl.: A61B 17/04

(54) **TISSUE ANCHORS WITH HEMOSTASIS FEATURES**
GEWEBEANKER MIT HÄMOSTASEMERKMALEN
ANCRES TISSULAIRES À FONCTIONS HÉMOSTATIQUES

(30) Priority: 31.10.2017 US 201762579281 P
(43) Date of publication of application: 10.06.2020
(73) Proprietor: 4Tech Inc., Waltham, MA 02452-8496 (US)
(72) Inventor: GRIFFIN, Patrick, Castlegar Galway (IE); DONNELLY, Evin, Salthill Galway (IE)
(74) Representative: Evens, Paul Jonathan
(86) International application number: PCT/US2018/056893
(87) International publication number: WO 2019/089262

(56) References cited:
- WO-A1-2014/164605
- WO-A1-2016/087934
- US-A1- 2002 013 571
- US-A1- 2006 142 797
- US-A1- 2008 071 310
- US-A1- 2014 100 604
- US-A1- 2015 238 340
- US-B1- 6 508 828

## Description

### CROSS-REFERENCE TO RELATED APPLICATIONS

The present application claims priority from US Provisional Patent Application 62/579,281 to Griffin et al., entitled, "Tissue anchors with hemostasis features," filed on October 31, 2017, which is assigned to the assignee of the present application.

### FIELD OF THE APPLICATION

The present invention relates generally to tissue anchors, and specifically to tissue anchors for implantation at cardiac sites.

### BACKGROUND OF THE APPLICATION

Tissue anchors are used for anchoring elements, such as pacemaker electrode leads or sutures, to tissue, such as bone or soft tissue. PCT Publication WO 2016/087934 to Gilmore et al., discloses a hemostatic tissue anchor according to the pre-amble of appended claim 1. The tissue anchor includes a shaft, a tissue-coupling element, and a flexible elongate tension member. The tissue-coupling element includes a wire, which is shaped as an open loop coil having, in some applications, more than one coil revolution when the tissue anchor is unconstrained, i.e., expanded from a linear state to a coiled state. The tension member includes a distal portion, that is fixed to a site on the open loop coil, a proximal portion, which has a longitudinal segment that runs alongside at least a portion of the shaft, and a crossing portion, which (i) is disposed between the distal and the proximal portions along the tension member, and (ii) crosses at least a portion of the open loop when the tissue anchor is expanded. The tissue anchor is configured to allow relative axial motion between the at least a portion of the shaft and the longitudinal segment of the proximal portion of the tension member when the tissue anchor is expanded. For some applications, a head of the tissue anchor is shaped so as to define a passage in which the proximal portion of the flexible elongate tension member is slidably disposed. The flexible elongate tension member comprises a locking stopper, which is axially fixed to the proximal or the crossing portion of the flexible elongate tension member. The locking stopper and the passage are sized and shaped such that the size and shape of the passage prevent proximal movement of the locking stopper past the passage. The locking stopper limits the total load that can be applied to the open loop by the flexible elongate tension member, thereby reducing excessive, unnecessary strain on the open loop. Additional load (tension) that is applied by the flexible elongate tension member pulls on the entire anchor, and does not further increase the load applied across the open loop.

US Patent 7,892,214 to Kagan et al. describes devices and methods for attachment of an endoluminal gastrointestinal device, such as an artificial stoma device, a gastrointestinal bypass sleeve device or an attachment cuff, within a patient's digestive tract for treatment of obesity.

US Patent 8,758,402 to Jenson et al. describes methods and devices for closing and/or sealing an opening in a vessel wall and/or an adjacent tissue tract. The '402 Patent describes a device for delivering and deploying an anchor, plug, suture, and/or locking element adjacent to the opening in the vessel wall and/or tissue tract.

US Patent 7,056,333 to Walshe describes a tissue-anchoring system, including a tissue-anchoring device and tissue anchors. The tissue-anchoring device includes a housing and a tissue anchor positioned on the tissue-anchoring device that is inserted into tissue or secured onto tissue. The device may optionally have a plunger assembly slidably positioned in the housing to assist advancing the anchor into a tissue. The tissue anchor has a barb end and a shaft. The barb end is adapted to resist removal from tissue after the anchor has been inserted. The tissue anchor shaft and the barb end may be hollow. The anchor shaft has an attachment member distal from the barb end for direct attachment to tissue or for attachment of sutures or slings. The tissue anchors may include an adjustment mechanism for intra- or post-operative adjustments.

US Patent Application Publication 2010/0217309 to Hansen et al. describes a modified arteriotomy closure plug, said modification selected from the group consisting of a slit, a notch, a groove, and combinations thereof and a method of manufacturing the arteriotomy closure plug.

US Patent Application Publication 2002/0013571 to Goldfarb et al. describes methods and devices for capturing and fixing leaflets in valve repair, and discloses a tissue anchor.

### SUMMARY OF THE APPLICATION

In accordance with the present invention, there is provided a hemostatis tissue anchor as defined in appended independent claim 1. Embodiments of the invention are defined in the appended claims dependent on independent claim 1. Methods of using the hemostatic tissue anchor are presented as a way to understand the invention and do not form part of the invention.

Embodiments of the present invention provide a hemostatic tissue anchor that is deliverable to a cardiac chamber in an unexpanded generally elongate configuration within a deployment tool. The hemostatic tissue anchor comprises an anchor portion that is configured to be anchored to a cardiac tissue wall at a target site such that a tensile force can be applied to the anchor portion and thus to the cardiac tissue wall, once the hemostatic tissue anchors are deployed, so as to draw tightly the anchor portion against the cardiac tissue wall at the target site.

The hemostatic tissue anchor comprises two or more discs that are configured to be disposed entirely within the cardiac tissue wall at the target site so as to act as a hemostatic seal of an opening through the cardiac tissue wall. For some applications, the two or more discs are soft and flexible. For some applications of the present invention, the two or more discs are rigid. For some applications of the present invention, each one of the two or more discs is tapered.

There is therefore provided, in accordance with some applications of the present invention, a hemostatic tissue anchor deliverable within a catheter to a target site, the hemostatic tissue anchor configured to be anchored to a cardiac tissue wall at the target site, the hemostatic tissue anchor including:
an anchor portion supported by a generally elongate anchor shaft releasably securable to the catheter, the anchor portion positioned at a distal end of the generally elongate anchor shaft, the anchor portion configured to expand from a first generally elongate configuration to a second expanded configuration such that the anchor portion in the second expanded configuration can be drawn tightly against the cardiac tissue wall at the target site when a tensile force is applied to the anchor portion; and
two or more discs coupled to the elongate anchor shaft, the two or more discs surrounding the elongate anchor shaft, the two or more discs being configured to be disposed entirely within the cardiac tissue wall at the target site,
whereby once the two or more discs are implanted entirely within the cardiac tissue wall at the target site, the two or more discs act as a hemostatic seal of an opening through the cardiac tissue wall, through which opening the elongate anchor shaft is disposed, characterized in that:
the hemostatic tissue anchor further comprises a sleeve surrounding at least a portion of the elongate anchor shaft, and wherein the two or more discs are integral with the sleeve and coupled to the elongate anchor shaft via the sleeve.

In some applications of the present invention, the two or more discs are soft and flexible.

In some applications of the present invention, the two or more discs are rigid.

In some applications of the present invention, the two or more discs include porous material.

In some applications of the present invention, the two or more discs include a bio-polymer.

In some applications of the present invention:
each one of the two or more discs is shaped so as to define a flat surface and a tapered surface,
the flat surface is closer to the anchor portion than the tapered surface is to the anchor portion, and
the tapered surface narrows in a proximal direction away from the flat surface.

In some applications of the present invention, the two or more discs include a material that is configured to elute a therapeutic agent.

In some applications of the present invention, the two or more discs are coated with a therapeutic agent.

In some applications of the present invention, the two or more discs include a hydrogel. In some applications of the present invention, the hydrogel includes a hydrogel that is configured to expand upon contact with fluid.

In some applications of the present invention, the two or more discs are arranged coaxially along the elongate anchor shaft.

In some applications of the present invention, the two or more discs are deformable when the hemostatic tissue anchor is disposed within the catheter for delivery to the target site.

In some applications, the cardiac tissue wall is a myocardial tissue wall, and the two or more discs are configured to be implanted entirely within the myocardial tissue wall. In some applications, the anchor portion is configured to be implanted in the pericardial cavity between visceral pericardium and parietal pericardium, generally alongside and against the parietal pericardium, without penetrating the parietal pericardium.

In some applications, the anchor portion, when expanded, defines a generally planar structure orthogonal to the elongate anchor shaft.

In some applications of the present invention, an anchor system is provided including the hemostatic tissue anchor, and the anchor system further includes a tether affixed to the hemostatic tissue anchor such that tensile force can be applied to the hemostatic tissue anchor via the tether. In some applications of the present invention, the hemostatic tissue anchor further includes an elongate tension member coupled to a portion of the anchor portion and the tether is affixed to the elongate tension member such that the tensile force can be applied to the anchor portion via the tether and the elongate tension member. In some applications of the present invention, the anchor system further includes a second tissue anchor separate and distinct from the hemostatic tissue anchor. In some applications of the present invention, the second tissue anchor is couplable to the hemostatic tissue anchor by the tether. In some applications of the present invention, the second tissue anchor is coupled to the hemostatic tissue anchor by the tether.

There is also provided a method for anchoring a hemostatic tissue anchor to a cardiac tissue wall at a target site, the method including:
delivering, to a cardiac chamber, the hemostatic tissue anchor within a catheter, the hemostatic tissue anchor configured to be anchored to a cardiac tissue wall at the target site and the hemostatic tissue anchor including:
   an anchor portion supported by a generally elongate anchor shaft releasably securable to the catheter, the anchor portion positioned at a distal end of the generally elongate anchor shaft, the anchor portion configured to expand from a first generally elongate configuration to a second expanded configuration such that the anchor portion in the second expanded configuration can be drawn tightly against the cardiac tissue wall at the target site when a tensile force is applied to the anchor portion; and
   one or more discs coupled to the elongate anchor shaft, the one or more discs surrounding the elongate anchor shaft, the one or more discs being configured to be disposed entirely within the cardiac tissue wall at the target site;
delivering the anchor portion in an unexpanded generally elongate configuration through the cardiac tissue wall from a first side of the wall to a second side of the wall, such that the anchor portion expands on the second side of the cardiac tissue wall, thereby anchoring the tissue anchor to the cardiac tissue wall at the target site; and
positioning the one or more discs entirely within the cardiac tissue wall at the target site whereby, once the one or more discs are implanted entirely within the cardiac tissue wall at the target site, the one or more discs act as a hemostatic seal of an opening through the cardiac tissue wall, through which opening the elongate anchor shaft is disposed.

In some applications, delivering the hemostatic tissue anchor includes delivering the hemostatic tissue anchor while the one or more discs are deformed within the catheter for deliver to the cardiac site.

In some applications, delivering the anchor portion in the unexpanded generally elongate configuration through the cardiac tissue wall includes delivering the anchor portion such that the anchor portion, when expanded, defines a generally planar structure orthogonal to the elongate anchor shaft.

In some applications, the cardiac tissue is a myocardial tissue wall, and delivering the anchor portion in the unexpanded generally elongate configuration through the cardiac tissue wall includes delivering the anchor portion through the cardiac tissue wall into the pericardial cavity between visceral pericardium and parietal pericardium, generally alongside and against the parietal pericardium, without penetrating the parietal pericardium.

In some applications, delivering the hemostatic tissue anchor includes delivering the hemostatic tissue anchor affixed to a tether, and the method further includes applying tensile force to the hemostatic tissue anchor via the tether.

In some applications, the cardiac tissue is a myocardial tissue wall, and wherein delivering the anchor portion in the unexpanded generally elongate configuration through the cardiac tissue wall includes delivering the anchor portion through the cardiac tissue wall into the pericardial cavity between visceral pericardium and parietal pericardium, generally alongside and against the parietal pericardium, without penetrating the parietal pericardium.

In some applications, applying the tensile force includes applying the tensile force to the myocardial tissue wall.

In some applications, delivering the hemostatic tissue anchor affixed to the tether includes delivering the hemostatic tissue anchor that includes an elongate tension member coupled to a portion of the anchor portion, the tether being affixed to the elongate tension member, and the method further includes applying the tensile force to the anchor portion via the tether and the elongate tension member.

In some applications, the method further includes implanting a second tissue anchor that is separate and distinct from the hemostatic tissue anchor.

In some applications, the method further includes coupling the tether to the hemostatic tissue anchor.

In some applications, the second tissue anchor is coupled to the hemostatic tissue anchor by the tether.

The present invention will be more fully understood from the following detailed description of embodiments thereof, taken together with the drawings, in which:

### BRIEF DESCRIPTION OF THE DRAWINGS

Fig. 1 is a schematic illustration of a hemostatic tissue anchor that is configured to be anchored to a cardiac tissue wall at a target site, in accordance with an application of the present invention;
Fig. 2 is a schematic illustration of the hemostatic tissue anchor of Fig. 1 deployed through a myocardial tissue wall, in accordance with an application of the present invention;
Fig. 3 is a schematic illustration of another hemostatic tissue anchor;
Fig. 4 is a schematic illustration of the hemostatic tissue anchor of Fig. 3 deployed through a myocardial tissue wall;
Fig. 5 is a schematic illustration of another hemostatic tissue anchor, in accordance with an application of the present invention;
Figs. 6A-6B are schematic illustration of the hemostatic tissue anchor of Fig. 5 deployed through a myocardial tissue wall, in accordance with an application of the present invention;
Fig. 7 is a schematic illustration of another hemostatic tissue anchor, in accordance with an application of the present invention; and
Fig. 8 is a schematic illustration of yet another hemostatic tissue anchor.

### DETAILED DESCRIPTION OF APPLICATIONS

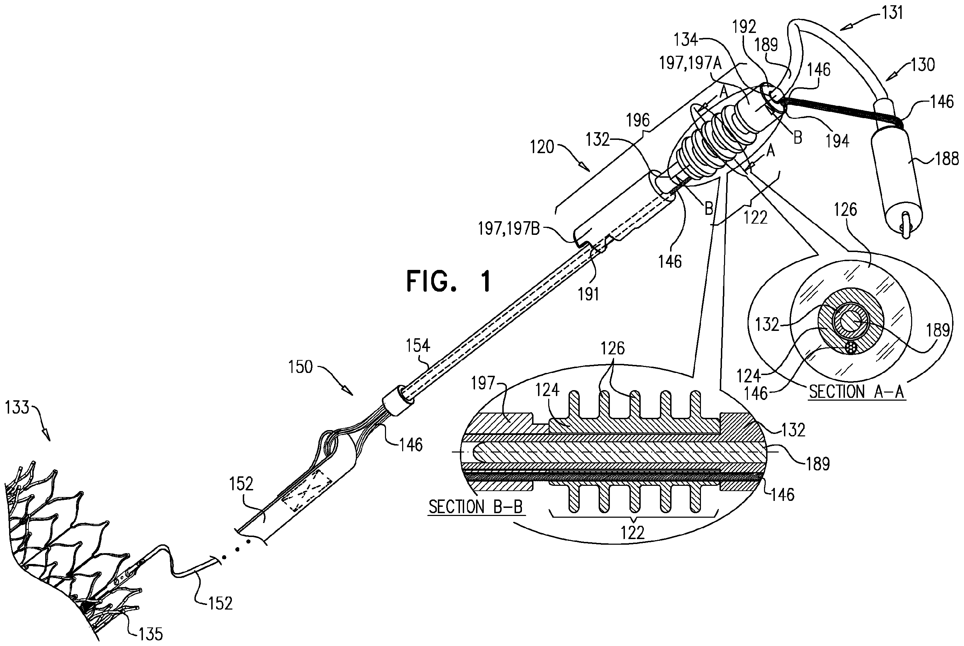

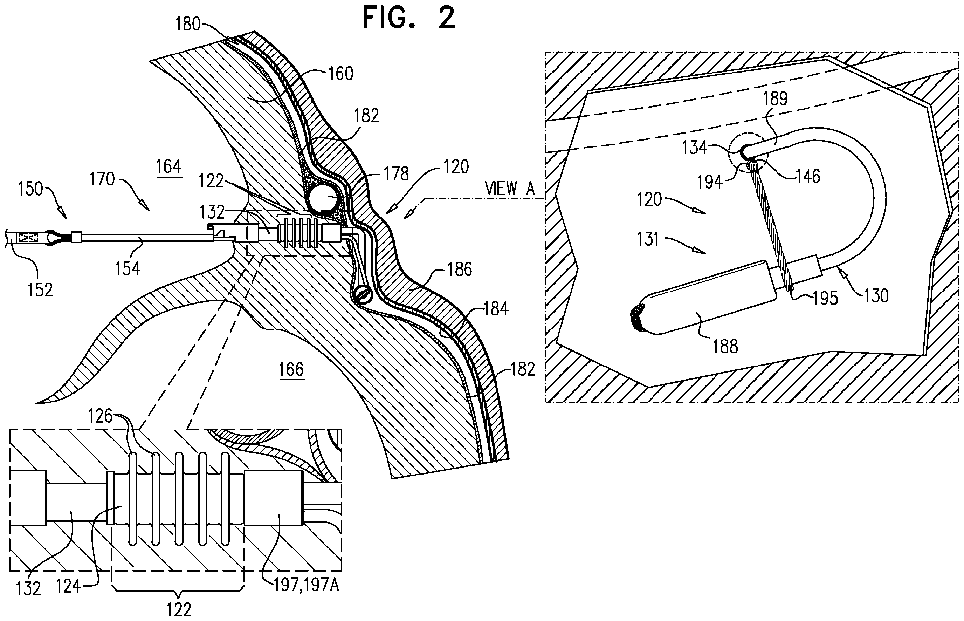

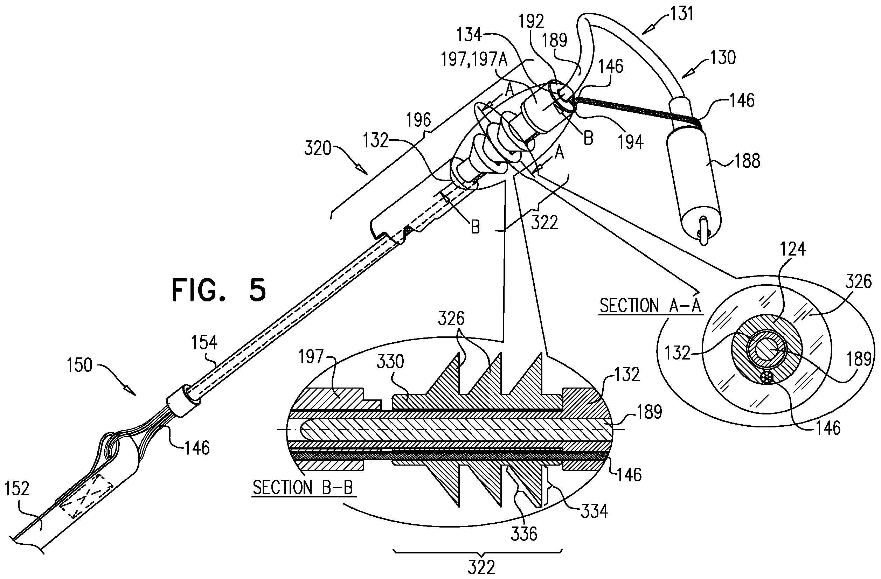

Fig. 1 is a schematic illustration of a hemostatic tissue anchor 120 that is configured to be anchored to a cardiac tissue wall at a target site, in accordance with an application of the present invention. Hemostatic tissue anchor 120 comprises an elongate anchor portion 130, which optionally is supported by an anchor head 196 at a proximal end 134 of anchor portion 130. Anchor portion 130 is configured to be delivered in an unexpanded generally elongate configuration through the cardiac tissue wall from a first side of the wall to a second side of the wall, such shown hereinbelow with reference to Fig. 2. Anchor portion 130 is further configured, upon deployment, to expand on the second side of the cardiac tissue wall, such as shown hereinbelow with reference to Fig. 2. Figs. 1 and 2 show anchor portion 130 expanded. For some applications, expanded anchor portion 130 has less than one turn, as shown in the figures, while for other applications, expanded anchor portion 130 has one turn (configuration not shown) or more than one turn (configuration not shown, but, for example, may be as shown in Figs. 5B-D, 6A-B, 7A-B, 9A-G, and/or 91 of above-mentioned PCT Publication WO 2016/087934).

Hemostatic tissue anchor 120 further comprises a flexible elongate tension member 146 coupled to a portion of anchor portion 130 of hemostatic tissue anchor 120. Through flexible elongate tension member 146, or components equivalent thereto, the tensile force can be applied to anchor portion 130 after it has been expanded. When applied in vivo, the tensile force may have the benefit of bringing the anchor close to the tissue wall to which it is applied. For some applications, an anchor system 150 is provided that comprises hemostatic tissue anchor 120 and a tether 152 affixed to flexible elongate tension member 146 such that the tensile force can be applied to hemostatic tissue anchor 120 via tether 152 and flexible elongate tension member 146. Optionally, hemostatic tissue anchor 120 further comprises a tube 154 that surrounds a proximal portion of flexible elongate tension member 146. For some applications, anchor system 150 further comprises a second tissue anchor 133, separate and distinct from hemostatic tissue anchor 120, such as is shown in above-mentioned PCT Publication WO 2016/087934. For some applications, the second tissue anchor, and additional anchors if so desired, is couplable or coupled to hemostatic tissue anchor 120 by one or more tethers that include tether 152.

Flexible elongate tension member 146 extends through a portion of (a) anchor portion 130 of hemostatic tissue anchor 120 and (b) a distal opening 194 of a passage 191 through hemostatic tissue anchor 120, such that expanded anchor portion 130 can be drawn tightly against the second side of the cardiac tissue wall at the target site when the tensile force is applied to anchor portion 130.

Distal opening 194 of passage 191 is typically located near (e.g., at) a distal end 192 of anchor head 196. A portion of flexible elongate tension member 146 is slidably disposed through passage 191. For some applications, passage 191 is defined by anchor head 196 (as shown). For example, distal opening 194 may be defined by a tubular anchor shaft 132 that anchor head 196 comprises; anchor head 196 may optionally implement techniques described in above-mentioned PCT Publication WO 2016/087934. For some applications, in addition to or instead of tubular anchor shaft 132, anchor head 196 comprises one or more collars 197, such as distal and proximal collars 197A and 197B, as shown, or exactly one collar 197 (configuration not shown). For some of these applications, distal opening 194 is defined by a distal end of distal collar 197A (as shown in Figs. 1 and 2) or a distal end of the exactly one collar 197 (configuration not shown). Passage 191 is typically a channel, but may also be a groove (e.g., a U-shaped groove).

For some applications, anchor portion 130, once expanded on the second side of the cardiac tissue wall, such as described hereinbelow with reference to Fig. 2, assumes a shape generally orthogonal to anchor head 196, as shown in Fig. 1, although it need not be orthogonal.

For some applications, as shown in Fig. 1, anchor head 196 further comprises a sealing element 122, which is sized and shaped to be inserted with anchor head 196 into an incision through the cardiac tissue wall. Sealing element 122, along with at least a portion of anchor head 196, remains in the incision upon completion of the implantation of hemostatic tissue anchor 120. Sealing element 122 promotes hemostasis to provide sealing of the incision. Sealing element 122 comprises two or more discs 126 surrounding shaft 132. Typically, discs 126 are soft and flexible and are configured to be disposed entirely within the cardiac tissue wall at the target site, as shown hereinbelow with reference to Fig. 2.

Anchor 120 is deliverable within a catheter (not shown) to a target site and is configured to be anchor to a cardiac tissue wall at the target site. Anchor portion 130 is supported by generally elongate anchor shaft 132 releasably securable to the catheter. Anchor portion 130 positioned at a distal end of generally elongate anchor shaft 132. Anchor portion 130 is configured to expand from a first generally elongate configuration to a second expanded configuration such that anchor portion 130 in the second expanded configuration can be drawn tightly against the cardiac tissue wall at the target site when a tensile force is applied to anchor portion 130.

As shown in Fig. 1, anchor 120 comprises a sleeve 124 surrounding at least a portion of elongate anchor shaft 132, and the two or more discs 126 are integral with sleeve 124 and coupled to elongate anchor shaft 132 via sleeve 124.

For some applications of the present invention discs 126 comprise porous material. For some applications, discs 126 comprise a bio-polymer. For some applications of the present invention, discs 126 comprise a material configured to elute a therapeutic agent. For some applications of the present invention, discs 126 are coated with a therapeutic agent. For applications in which discs 126 are configured to elute a therapeutic agent or are coated with a therapeutic agent, the therapeutic agent comprises, for example, a fibrosis-enhancing drug, an agent which promotes tissue growth, a clotting agent, an anti-inflammatory, and/or an antibiotic. For some applications, discs 126 comprise a hydrogel. For some applications of the present invention in which discs 126 comprise the hydrogel, the nature and composition of the hydrogel is such that it expands and/or swells upon contact with blood or fluid in order to increase the diameter of discs 126, thereby increasing surface area interaction between discs 126 and surrounding tissue as well as improving the overall hemostatic effect of discs 126. The two or more discs 126 may comprise 3 or more discs or 5 or more discs, such as exactly 5 discs, as shown in Figs. 1 and 2 by way of illustration and not limitation. As shown, the two or more discs 126 are arranged coaxially along elongate anchor shaft 132.

Typically, discs 126 are deformable when hemostatic tissue anchor 120 is disposed within the catheter for delivery to the target site.

For some applications, sleeve 124 and discs 126 are fabricated from a single piece. For other applications, sleeve 124 and discs 126 are fabricated individually and subsequently fixed together during assembly of anchor 120.

For some applications, sleeve 124 and discs 126 are axially compressible and expandable.

For some application, hemostatic tissue anchor 120 defines a first tissue anchor 131 and anchor system 150 comprises a second tissue anchor 133 separate and distinct from hemostatic tissue anchor 120. As shown, second tissue anchor 133 is couplable to hemostatic tissue anchor 120 by tether 152. For some applications of the present invention, second tissue anchor 133 is coupled to hemostatic tissue anchor 120 by tether 152. For some applications, such as shown in Fig. 1, second tissue anchor 133 comprises a stent 135. For example, second tissue anchor 133 may implement techniques described in one or more of the following applications: US Patent Application Publication 2011/0184510, US Patent Application Publication 2012/0035712, US Patent Application Publication 2013/0018459, US Patent Application Publication 2013/0046380, and/or PCT Publication WO 2014/141239. It is to be noted that for some applications of the present invention, second tissue anchor 133 may comprise any suitable tissue anchor known in the art, e.g., a helical anchor. It is to be noted that any suitable number of tissue anchors may be coupled or couplable to hemostatic tissue anchor 120 by one or more tethers that include tether 152.

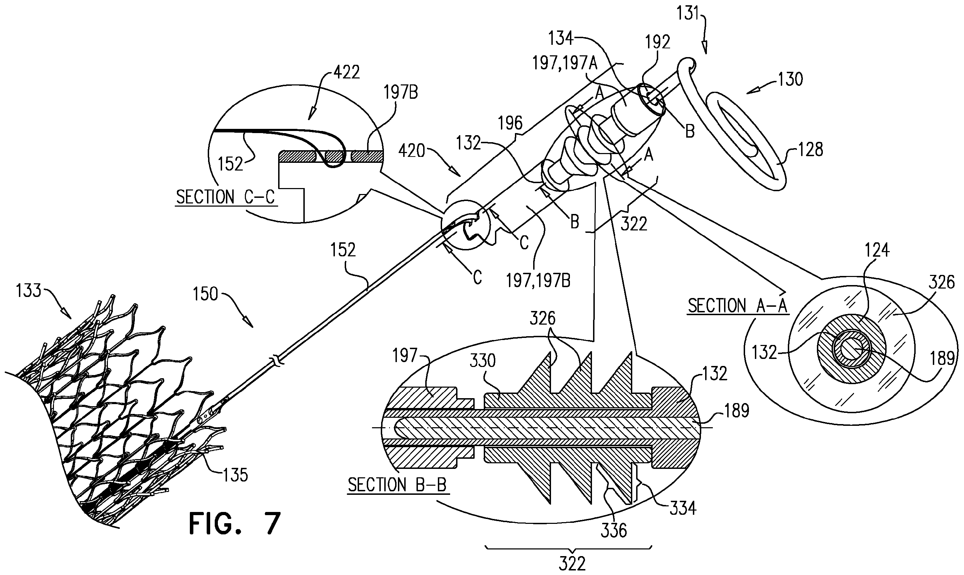

It is to be noted that hemostatic tissue anchor 120 may be provided independently of flexible elongate tension member 146, as is described hereinbelow with reference to Fig. 7.

Reference is now made to Fig. 2, which is a schematic illustration of hemostatic tissue anchor 120 deployed through a myocardial tissue wall 60, in accordance with an application of the present invention. Although in Fig. 2 hemostatic tissue anchor 120 is shown deployed through a myocardial tissue wall, hemostatic tissue anchor 120 may also be deployed through other cardiac tissue walls, such as the interatrial septum, either at or not at the fossa ovalis, or through other non-cardiac tissue walls. Indeed, the tissue anchors described herein may be deployed in any number of bodily locations where it is desired to anchor into or behind tissue for purposes of moving such tissue relative to adjacent tissue.

Typically, but not necessarily, hemostatic tissue anchor 120 is delivered to a target site, such as a cardiac chamber, in an unexpanded generally elongate configuration within a deployment tool 170, which may comprise a catheter. During the delivery, discs 126 are deformable within tool 170 (e.g., within the catheter) for delivery to the target site. The cardiac chamber may be a right atrium 164 (as shown), a right ventricle 166 (configuration not shown), a left atrium (configuration not shown), or a left ventricle (configuration not shown). In one application, a hollow needle (not shown) is used to puncture through a first side of a myocardial tissue wall 160 and visceral pericardium 182 (which is part of the epicardium), avoiding vasculature such as the right coronary artery (RCA) 178. For some applications, deployment tool 170 is then further directed into the pericardial cavity 180 between visceral pericardium 182 and parietal pericardium 184, carefully avoiding puncturing parietal pericardium 184 and fibrous pericardium 186.

As shown, anchor portion 130 expands on the second side of myocardial tissue wall 160, thereby anchoring hemostatic tissue anchor 120 to myocardial tissue wall 160. Anchor portion 130 is shaped as an open loop coil having, in some applications, more than one coil revolution when the tissue anchor is unconstrained, i.e., expanded from a linear state to a coiled state. That is, when anchor portion 130 expands from a first generally elongate configuration to a second expanded configuration, portion 130 recovers to a generally coiled configuration, as shown. Anchor portion 130, when expanded, defines a generally planar structure orthogonal to the elongate anchor shaft 132.

Once hemostatic tissue anchor 120 has been anchored to myocardial tissue wall 160 at the target site, expanded anchor portion 130 is tightly drawn against the second side of myocardial tissue wall 160 at the target site by applying a tensile force, using tether 152, to anchor portion 130 and thus to myocardial tissue wall 160. Application of the tensile force partially compresses expanded anchor portion 130.

For some applications, after application of the tensile force, all or a portion of anchor portion 130 rests against the second side of myocardial tissue wall 160, generally helping prevent possible inadvertent cutting of myocardial tissue wall 160 by flexible elongate tension member 146. For some applications, anchor portion 130 is delivered through myocardial tissue wall 160, into pericardial cavity 180, generally alongside and against parietal pericardium 184, without penetrating the parietal pericardium 184. For some applications, a second tissue anchor is implanted, separate and distinct from hemostatic tissue anchor 120.

Reference is made to Figs. 1 and 2. For some applications, anchor portion 130 comprises a tip 188, which is fixed to a distal end of a wire 189 of anchor portion 130. Tip 188, at a widest longitudinal site along tip 188, has a greatest outer cross-sectional area that equals at least 150% (e.g., at least 200%, or at least 300%) of an average cross-sectional area of wire 189. (The cross-sectional area of tip 188 is measured perpendicular to a central longitudinal axis of tip 188. Similarly, the cross-sectional area of wire 189 is measured perpendicular to a central longitudinal axis of the wire, and is not a cross-sectional area of anchor portion 130.) Tip 188 temporarily serves as an atraumatic distal end of the hollow needle of deployment tool 170 when the tip is removably coupled to a distal end of the hollow needle. Wire 189 may be solid or hollow (i.e., tubular).

As shown in Fig. 2, discs 126 are implanted entirely within myocardial tissue wall 160 (e.g., within the myocardial tissue wall) and act as a hemostatic seal of an opening through myocardial tissue wall 160, through which opening elongate anchor shaft 132 is disposed.

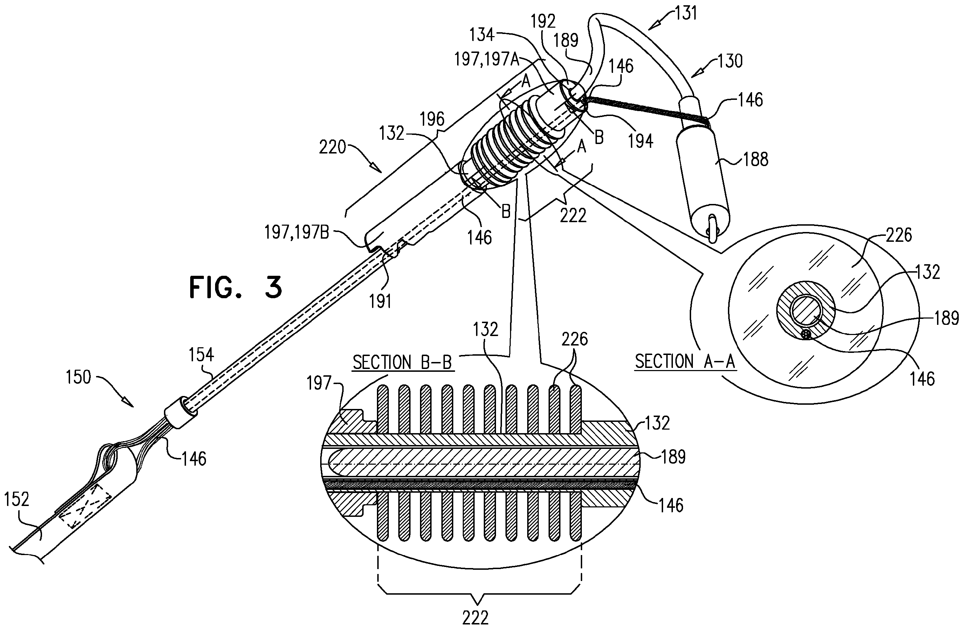

Reference is now made to Figs. 3-4, which are schematic illustrations of a hemostatic tissue anchor 220. Except as described below, hemostatic tissue anchor 220 is generally similar to hemostatic tissue anchor 120, described hereinabove with reference to Figs. 1 and 2 and like reference numerals refer to like parts.

Anchor 220 is deliverable within a catheter (not shown) to a target site and is configured to be anchor to a cardiac tissue wall at the target site. Anchor portion 130 is supported by generally elongate anchor shaft 132 releasably securable to the catheter. Anchor portion 130 positioned at a distal end of generally elongate anchor shaft 132. Anchor portion 130 is configured to expand from a first generally elongate configuration to a second expanded configuration such that anchor portion 130 in the second expanded configuration can be drawn tightly against the cardiac tissue wall at the target site when a tensile force is applied to anchor portion 130.

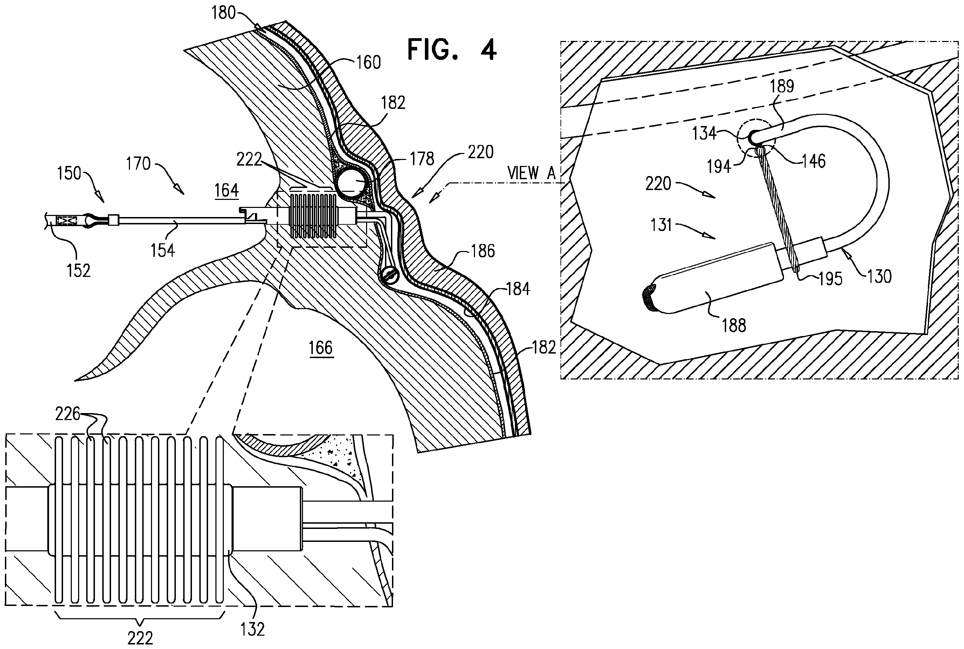

Anchor head 196 of anchor 220 comprises a sealing element 222, which is sized and shaped to be inserted with anchor head 196 into an incision through the cardiac tissue wall. Sealing element 222, along with at least a portion of anchor head 196, remains in the incision upon completion of the implantation of hemostatic tissue anchor 220. Sealing element 222 promotes hemostasis to provide sealing of the incision. For some applications, sealing element 222 comprises one or more discs 226 surrounding shaft 132. Typically, discs 226 are soft and flexible and are configured to be disposed entirely within the cardiac tissue wall at the target site, as shown in Fig. 4.

Discs 226 are directly coupled to shaft 132 and, unlike anchor 120 of Figs. 1-2, anchor 220 does not comprise sleeve 124.

For some applications, discs 226 comprise porous material. For some applications, discs 226 comprise a bio-polymer. For some applications, discs 226 comprise a material configured to elute a therapeutic agent. For some applications of the present invention, discs 226 are coated with a therapeutic agent. For applications in which discs 226 are configured to elute a therapeutic agent or are coated with a therapeutic agent, the therapeutic agent comprises, for example, a fibrosis-enhancing drug, an agent which promotes tissue growth, a clotting agent, an anti-inflammatory, and/or an antibiotic. For some applications, discs 226 comprise a hydrogel. For some applications in which discs 226 comprise the hydrogel, the nature and composition of the hydrogel is such that it expands and/or swells upon contact with blood or fluid in order to increase the diameter of discs 226, thereby increasing surface area interaction between discs 226 and surrounding tissue as well as improving the overall hemostatic effect of discs 226. Typically, one or more discs 226 comprise two or more discs (e.g., 3 or more discs or 5 or more discs, such as exactly 11 discs, as shown in Figs. 3 and 4 by way of illustration and not limitation). As shown, the two or more discs 226 are arranged coaxially along elongate anchor shaft 132.

Typically, discs 226 are deformable when hemostatic tissue anchor 220 is disposed within the catheter for delivery to the target site.

As shown in Fig. 4, discs 226 are implanted entirely within myocardial tissue wall 160 (e.g., within the myocardial tissue wall) and act as a hemostatic seal of an opening through myocardial tissue wall 160, through which opening elongate anchor shaft 132 is disposed.

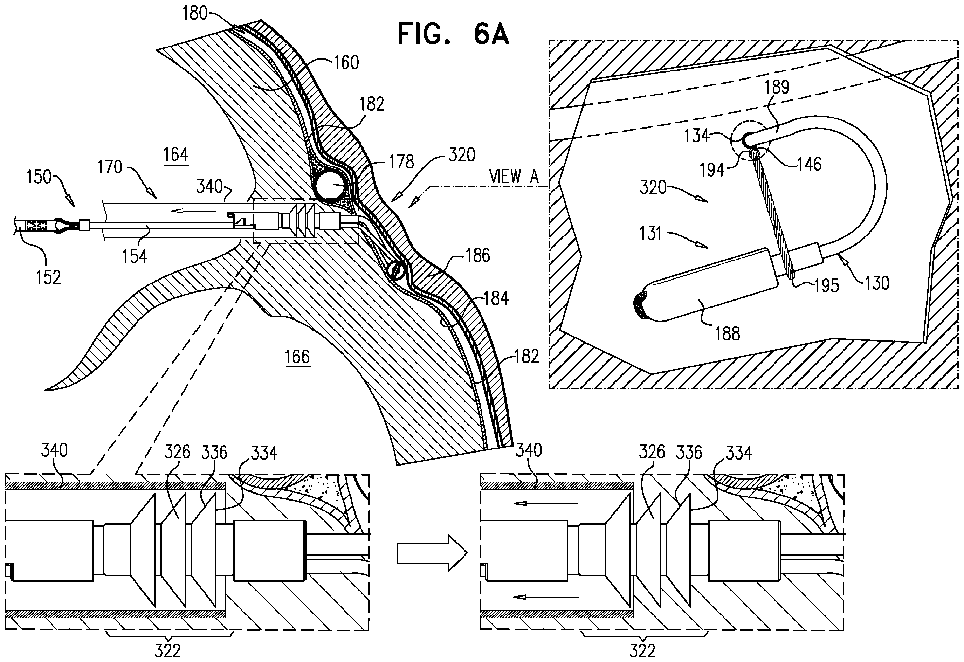

Reference is now made to Figs. 5 and 6A-B, which are schematic illustrations of a hemostatic tissue anchor 320, in accordance with some applications of the present invention. Except as described below, hemostatic tissue anchor 320 is generally similar to hemostatic tissue anchor 120, described hereinabove with reference to Figs. 1 and 2 and like reference numerals refer to like parts.

Anchor 320 is deliverable within a catheter (not shown) to a target site and is configured to be anchor to a cardiac tissue wall at the target site. Anchor portion 130 is supported by generally elongate anchor shaft 132 releasably securable to the catheter. Anchor portion 130 positioned at a distal end of generally elongate anchor shaft 132. Anchor portion 130 is configured to expand from a first generally elongate configuration to a second expanded configuration such that anchor portion 130 in the second expanded configuration can be drawn tightly against the cardiac tissue wall at the target site when a tensile force is applied to anchor portion 130.

Anchor head 196 of anchor 320 comprises a sealing element 322, which is sized and shaped to be inserted with anchor head 196 into an incision through the cardiac tissue wall. Sealing element 322, along with at least a portion of anchor head 196, remains in the incision upon completion of the implantation of hemostatic tissue anchor 320. Sealing element 322 promotes hemostasis to provide sealing of the incision. Sealing element 322 comprises two or more discs 326 surrounding shaft 132. Typically, discs 326 are rigid and are configured to be disposed entirely within the cardiac tissue wall at the target site, as shown in Figs. 6A-B.

As shown, discs 326 are coupled to a collar 330 which surrounds shaft 132.

Each disc 326 is shaped to as to define a flat surface 334 at a distal end thereof and a tapered surface 336 at a proximal end thereof. Flat surface 334 is closer to anchor portion 130 than tapered surface 336 is to anchor portion 130. Tapered surface 336 narrows in a proximal direction away from flat surface 334 along a longitudinal axis of anchor 320. The function of each of surfaces 334 and 336 is described hereinbelow with reference to Figs. 6A-B.

For some applications, discs 326 comprise a bio-polymer. For some applications of the present invention, discs 326 comprise a material configured to elute a therapeutic agent. For some applications of the present invention, discs 326 are coated with a therapeutic agent. For applications in which discs 326 are configured to elute a therapeutic agent or are coated with a therapeutic agent, the therapeutic agent comprises, for example, a fibrosis-enhancing drug, an agent which promotes tissue growth, a clotting agent, an anti-inflammatory, and/or an antibiotic. The two or more discs 326 may comprise 3 or more discs or 5 or more discs, such as exactly 3 discs, as shown in Figs. 5 and 6A-B by way of illustration and not limitation. As shown, the two or more discs 326 are arranged coaxially along elongate anchor shaft 132.

Typically, discs 326 are rigid. For some applications, discs 326 are soft and flexible and are deformable when hemostatic tissue anchor 320 is disposed within the catheter for delivery to the target site.

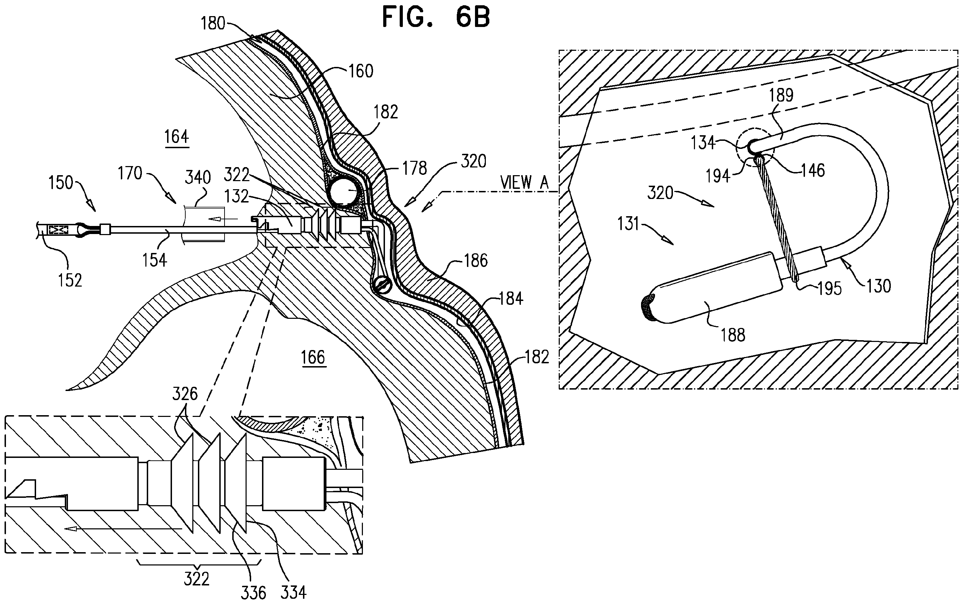

As shown in Fig. 6B, discs 326 are implanted entirely within myocardial tissue wall 160 (e.g., within the myocardial tissue wall) and act as a hemostatic seal of an opening through myocardial tissue wall 160, through which opening elongate anchor shaft 132 is disposed.

As shown in Fig. 6A, anchor 320 is delivered to cardiac tissue while discs 326 are protected within an overtube 340. As anchor 320 is delivered, overtube 340 pushes away cardiac tissue laterally from the longitudinal axis of anchor 320. Once anchor 320 is properly positioned within the cardiac tissue in a manner in which sealing element 322 is disposed entirely within the cardiac tissue, overtube 340 is gradually retracted proximally (as shown in Fig. 6A) so as to expose discs 326. Typically, since discs 326 are rigid, as discs 326 are exposed from within overtube 340, the cardiac tissue closes around the discs 326 and, thereby, discs 326 function as a hemostatic seal.

Once sealing element 322 is appropriately positioned within the cardiac tissue, as shown in Fig. 6B, tensile force can be applied to hemostatic tissue anchor 320 via tether 152 and flexible elongate tension member 146. Tapered surfaces 336 of discs 326 accommodates the tensile force tether 152 may have on the cardiac tissue during the application of tensile force to hemostatic tissue anchor 320 via tether 152. It is to be noted that most of the tensile force, however, is applied to anchor portion 130 via tension member 146. Tapered surfaces 336 are so tapered so as to provide an atraumatic interface between discs 326 and surrounding cardiac tissue, in particular, during the application of the tensile forces by tether 152. Flat surfaces 334 of discs 326 prevent distal migration of anchor 320. In particular, flat surfaces 334 of discs 326 prevent distal migration of sealing element 322 toward pericardial cavity 180. Such distal migration of sealing element 322 may occur responsively to the tensile force applied by anchor portion 130 to the proximal portions of anchor 320 in the proximal direction once tether 152 has applied tension to anchor portion 130.

Reference is now made to Figs. 1 and 5-6B. It is to be noted that the scope of the present invention includes anchor systems 150 of Figs. 5-6B comprising a second tissue anchor 133, as described hereinabove with reference to Fig. 1.

Reference is now made to Fig. 7, which is a schematic illustration of a hemostatic tissue anchor 420, in accordance with some applications of the present invention. Except as described below, hemostatic tissue anchor 420 is generally similar to hemostatic tissue anchor 320, described hereinabove with reference to Figs. 5 and 6A-B, and like reference numerals refer to like parts. As shown, anchor portion 130 comprises a tissue-coupling element 128, which extends from a distal end of shaft 132. For some applications, shaft 132 and tissue-coupling element 128 are integral to one another; for example, shaft 132 and tissue-coupling element 128 may comprise a wire.

For some applications, one or more tethers 152 are provided, which are configured to be coupled to tissue anchor 420, such as to anchor head 196 (e.g., to collars 197, such as to proximal collar 197B, as shown); for example, one of the one or more tethers 152 may be fixed to head collar 197B. As shown in Section C-C, proximal collar 197B is shaped so as to define a coupling 422 around which a portion of tether 152 is looped in order to affix and couple tether 152 to hemostatic tissue anchor 420 such that tensile force can be applied to hemostatic tissue anchor 420 via tether 152. In such a configuration, a portion of tether 152 is looped with respect to coupling 422 in order to affix and directly couple tether 152 to hemostatic tissue anchor 420. For such applications, an anchor system 150 is provided that comprises hemostatic tissue anchor 420 and tether 152 affixed to anchor 420 such that the tensile force can be applied directly to hemostatic tissue anchor 420 via tether 152.

For some application, hemostatic tissue anchor 420 defines a first tissue anchor 131 and anchor system 150 comprises a second tissue anchor 133 separate and distinct from hemostatic tissue anchor 420. As shown, second tissue anchor 133 is couplable to hemostatic tissue anchor 420 by tether 152. For some applications of the present invention, second tissue anchor 133 is coupled to hemostatic tissue anchor 420 by tether 152. For some applications, such as shown in Fig. 1, second tissue anchor 133 comprises a stent 135. For example, second tissue anchor 133 may implement techniques described in one or more of the following applications: US Patent Application Publication 2011/0184510, US Patent Application Publication 2012/0035712, US Patent Application Publication 2013/0018459, US Patent Application Publication 2013/0046380, and/or PCT Publication WO 2014/141239. It is to be noted that for some applications of the present invention, second tissue anchor 133 may comprise any suitable tissue anchor known in the art, e.g., a helical anchor. It is to be noted that any suitable number of tissue anchors may be coupled or couplable to hemostatic tissue anchor 420 by one or more tethers that include tether 152.

For some applications, a deployment tool (not shown) is used to constrain tissue-coupling element 128 while delivering tissue-coupling element 128 through tissue. Typically, during delivery, the deployment tool is configured to hold tissue-coupling element 128 in an elongated configuration, which may be straight or curvy. For some applications, the deployment tool comprises a shaft shaped so as to define a lumen, such as a hypodermic needle. The lumen is sized to hold tissue-coupling element 128 constrained therein, and, optionally, to hold other portions of tissue anchor 420 therein, such as shaft 132 and/or head 196.

For some applications, tissue-coupling element 128 is fixed to a distal end of a wire 189 of anchor portion 130.

Fig. 7 shows tissue-coupling element 128 of tissue anchor 420 unconstrained by a deployment tool. For some applications, anchor portion 130, once expanded on the second side of the cardiac tissue wall, assumes a shape generally orthogonal to anchor head 196, although it need not be orthogonal. Tissue-coupling element 128 is configured to have a predetermined shape when unconstrained by deployment tool 30. For example, tissue-coupling element 128 may comprise a shape-memory material, such as a shape-memory alloy, e.g., Nitinol. Thus, tissue-coupling element 128 automatically transitions to the predetermined shape when released from being constrained by the deployment tool to being unconstrained by the deployment tool.

It is to be noted that tissue-coupling element 128 may be similar or identical to tissue-coupling element 128 described in above-mentioned PCT Publication WO 2016/087934.

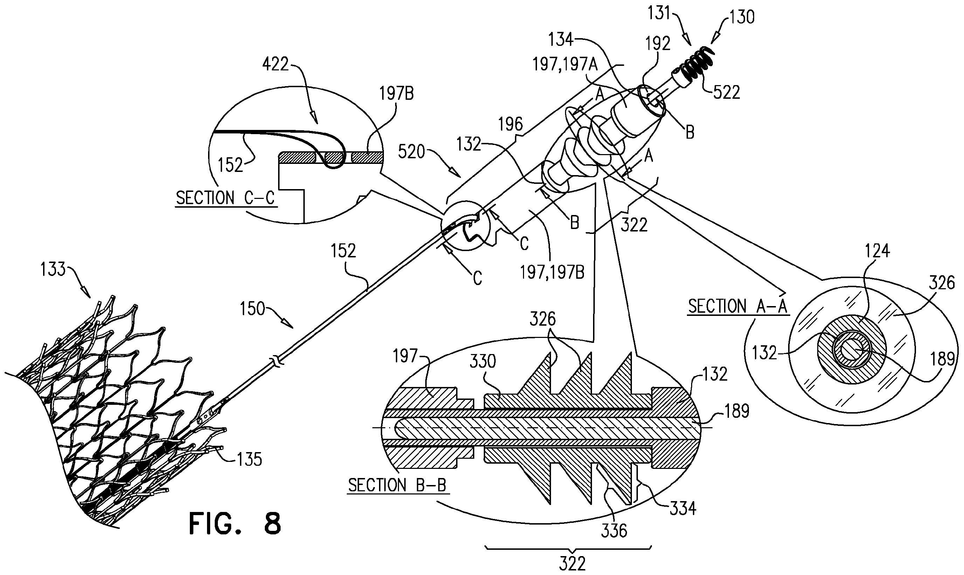

Reference is now made to Fig. 8, which is a schematic illustration of a hemostatic tissue anchor 520. Except as described below, hemostatic tissue anchor 520 is generally similar to hemostatic tissue anchor 420, described hereinabove with reference to Fig. 7, and like reference numerals refer to like parts. As shown, anchor portion 130 comprises a helical tissue anchor 522, which extends from a distal end of shaft 132. For some applications, shaft 132 and helical tissue anchor 522 are integral to one another; for example, shaft 132 and helical tissue anchor 522 may comprise a wire.

Reference is now made to Figs. 1-8. It is to be noted that anchor system 150 and hemostatic tissue anchors described herein with reference to Figs. 1-6B may be provided independently of flexible elongate tension member 146, as is described hereinabove with reference to Figs. 7 and 8. In such applications, tether 152 is affixed to the hemostatic tissue anchor such that tensile force can be applied to the hemostatic tissue anchor via the tether.

Reference is again made to Figs. 1-8. It is to be noted that for some applications, first and second tissue anchors 131 and 133 may comprise any suitable tissue anchor known in the art, e.g., a helical anchor. It is to be further noted that any suitable number of tissue anchors may be coupled or couplable to hemostatic tissue anchor 120 by one or more tethers that include tether 152.

Reference is yet again made to Figs. 1-8. It is to be noted that apparatus and methods described herein may be used to repair a cardiac valve of a patient. For example, apparatus and methods described herein may be used to repair a tricuspid valve of a patient using techniques described in above-mentioned PCT Publication WO 2016/087934.

The scope of the present invention is defined by the appended claims. The following applications, which are assigned to the assignee of the present application, disclose techniques and apparatus of the prior art: US Patent 8,475,525 to Maisano et al.; US Patent 8,961,596 to Maisano et al.; US Patent 8,961,594 to Maisano et al.; PCT Publication WO 2011/089601; US Patent 9,241,702 to Maisano et al.; US Provisional Application 61/750,427, filed January 9, 2013; US Provisional Application 61/783,224, filed March 14, 2013; US Provisional Application 61/897,491, filed October 30, 2013; US Provisional Application 61/897,509, filed October 30, 2013; US Patent 9,307,980 to Gilmore et al.; PCT Publication WO 2014/108903; PCT Publication WO 2014/141239; US Provisional Application 62/014,397, filed June 19, 2014; PCT Publication WO 2015/063580; US Patent Application Publication 2015/0119936; US Provisional Application 62/086,269, filed December 2, 2014; US Provisional Application 62/131,636, filed March 11, 2015; US Provisional Application 62/167,660, filed May 28, 2015; PCT Publication WO 2015/193728; PCT Publication WO 2016/087934; US Patent Application Publication 2016/0235533; US Patent Application Publication 2016/0242762; PCT Publication WO 2016/189391; US Patent Application Publication 2016/0262741; US Provisional Application 62/376,685, filed August 18, 2016; US Provisional Application 62/456,206, filed February 8, 2017; US Provisional Application 62/456,202, filed February 8, 2017; US Provisional Application 62/465,410, filed March 1, 2017; and US Provisional Application 62/465,400, filed March 1, 2017.

It will be appreciated by persons skilled in the art that the present invention is not limited to what has been particularly shown and described hereinabove. Rather, the scope of the present invention is defined by the appended claims, and may include variations and modifications thereof which would occur to persons skilled in the art upon reading the foregoing description.

## Claims

1. A hemostatic tissue anchor (120, 320, 420) deliverable within a catheter to a target site, the hemostatic tissue anchor (120, 320, 420) configured to be anchored to a cardiac tissue wall at the target site, the hemostatic tissue anchor (120, 320, 420) comprising:
an anchor portion (130) supported by a generally elongate anchor shaft (132) releasably securable to the catheter, the anchor portion (130) positioned at a distal end of the generally elongate anchor shaft (132), the anchor portion (130) configured to expand from a first generally elongate configuration to a second expanded configuration such that the anchor portion (130) in the second expanded configuration can be drawn tightly against the cardiac tissue wall at the target site when a tensile force is applied to the anchor portion (130); and
two or more discs (126, 326) coupled to the elongate anchor shaft (132), the two or more discs (126, 326) surrounding the elongate anchor shaft (132), the two or more discs (126, 326) being configured to be disposed entirely within the cardiac tissue wall at the target site,
whereby, once the two or more discs (126, 326) are implanted entirely within the cardiac tissue wall at the target site, the two or more discs (126, 326) act as a hemostatic seal of an opening through the cardiac tissue wall, through which opening the elongate anchor shaft (132) is disposed, **characterised in that**:
the hemostatic tissue anchor (120, 320, 420) further comprises a sleeve (124, 330) surrounding at least a portion of the elongate anchor shaft (132), and wherein the two or more discs (126, 326) are integral with the sleeve (124, 330) and coupled to the elongate anchor shaft (132) via the sleeve (124, 330).

2. The hemostatic tissue anchor according to Claim 1, wherein the two or more discs (126, 326) are soft and flexible.

3. The hemostatic tissue anchor according to Claim 1, wherein the two or more discs (326) are rigid.

4. The hemostatic tissue anchor according to any one of Claims 1-3, wherein the two or more discs (126) comprise porous material.

5. The hemostatic tissue anchor according to any one of Claims 1-3, wherein the two or more discs (126, 326) comprise a bio-polymer.

6. The hemostatic tissue anchor according to any one of Claims 1-3, wherein:
each one of the two or more discs (326) is shaped so as to define a flat surface (334) and a tapered surface (336),
the flat surface (334) is closer to the anchor portion (130) than the tapered surface (336) is to the anchor portion (130), and
the tapered surface (336) narrows in a proximal direction away from the flat surface (334).

7. The hemostatic tissue anchor according to any one of Claims 1-3, wherein the two or more discs (126, 326) comprise a material that is configured to elute a therapeutic agent, or the two or more discs (126, 326) are coated with a therapeutic agent.

8. The hemostatic tissue anchor according to any one of Claims 1-3, wherein the two or more discs (126) comprise a hydrogel.

9. The hemostatic tissue anchor according to any one of Claims 1-3, wherein the two or more discs (126, 326) comprise three or more discs (126, 326).

10. The hemostatic tissue anchor according to Claim 9, wherein the three or more discs (126, 326) are arranged coaxially along the elongate anchor shaft (132).

11. The hemostatic tissue anchor according to any one of Claims 1-2, wherein the two or more discs (126, 326) are deformable when the hemostatic tissue anchor (120, 320, 420) is disposed within the catheter for delivery to the target site.

12. The hemostatic tissue anchor according to any one of Claims 1-3, wherein the cardiac tissue wall is a myocardial tissue wall, and wherein the two or more discs (126, 326) are configured to be implanted entirely within the myocardial tissue wall.

13. An anchor system (150) comprising the hemostatic tissue anchor (120, 320, 420) according to any one of Claims 1-3, wherein the anchor system (150) further comprises a tether (152) affixed to the hemostatic tissue anchor (120, 320, 420) such that tensile force can be applied to the hemostatic tissue anchor (120, 320, 420) via the tether (152).

14. The anchor system according to Claim 13, wherein the hemostatic tissue anchor (120, 320, 420) further comprises an elongate tension member (146) coupled to a portion of the anchor portion (130) and wherein the tether (152) is affixed to the elongate tension member (146) such that the tensile force can be applied to the anchor portion (130) via the tether (152) and the elongate tension member (146).

15. The anchor system according to Claim 13, further comprising a second tissue anchor (133) separate and distinct from the hemostatic tissue anchor (120, 320, 420), wherein the second tissue anchor (133) is couplable to or coupled to the hemostatic tissue anchor (120, 320, 420) by the tether (152).

## Patentansprüche

1. Hämostatischer Gewebeanker (120, 320, 420), der innerhalb eines Katheters an eine Zielstelle abgegeben werden kann, wobei der hämostatische Gewebeanker (120, 320, 420) konfiguriert ist, um an einer Herzgewebewand an der Zielstelle verankert zu werden, wobei der hämostatische Gewebeanker (120, 320, 420) umfasst:
einen Ankerabschnitt (130), der von einer allgemein länglichen Ankerwelle (132) getragen wird, die lösbar an dem Katheter befestigt werden kann, wobei der Ankerabschnitt (130) an einem distalen Ende der allgemein länglichen Ankerwelle (132) positioniert ist, wobei der Ankerabschnitt (130) konfiguriert ist, um sich von einer ersten allgemein länglichen Konfiguration zu einer zweiten ausgedehnten Konfiguration so auszudehnen, dass der Ankerabschnitt (130) in der zweiten ausgedehnten Konfiguration an der Zielstelle fest an die Herzgewebewand gezogen werden kann, wenn eine Zugkraft auf den Ankerabschnitt (130) ausgeübt wird; und
zwei oder mehr Scheiben (126, 326), die mit der länglichen Ankerwelle (132) verbunden sind, wobei die zwei oder mehr Scheiben (126, 326) die längliche Ankerwelle (132) umgeben, wobei die zwei oder mehr Scheiben (126, 326) konfiguriert sind, um vollständig innerhalb der Herzgewebewand an der Zielstelle angeordnet zu sein,
wobei, sobald die zwei oder mehr Scheiben (126, 326) vollständig innerhalb der Herzgewebewand an der Zielstelle implantiert sind, die zwei oder mehr Scheiben (126, 326) als hämostatische Abdichtung einer Öffnung durch die Herzgewebewand wirken, durch welche Öffnung die längliche Ankerwelle (132) angeordnet ist, **dadurch gekennzeichnet, dass**:
der hämostatische Gewebeanker (120, 320, 420) ferner eine Hülse (124, 330) umfasst, die mindestens einen Abschnitt des länglichen Ankerschafts (132) umgibt, und wobei die zwei oder mehr Scheiben (126, 326) einstückig mit der Hülse (124, 330) sind und über die Hülse (124, 330) mit der länglichen Ankerwelle (132) verbunden sind.

2. Hämostatischer Gewebeanker nach Anspruch 1, wobei die zwei oder mehr Scheiben (126, 326) weich und flexibel sind.

3. Hämostatischer Gewebeanker nach Anspruch 1, wobei die zwei oder mehr Scheiben (326) starr sind.

4. Hämostatischer Gewebeanker nach einem der Ansprüche 1 bis 3, wobei die zwei oder mehr Scheiben (126) poröses Material umfassen.

5. Hämostatischer Gewebeanker nach einem der Ansprüche 1 bis 3, wobei die zwei oder mehr Scheiben (126, 326) ein Biopolymer umfassen.

6. Hämostatischer Gewebeanker nach einem der Ansprüche 1 bis 3, wobei: jede der zwei oder mehr Scheiben (326) so geformt ist, dass sie eine flache Oberfläche (334) und eine sich verjüngende Oberfläche (336) definiert,
die flache Oberfläche (334) näher an dem Ankerabschnitt (130) ist als die sich verjüngende Oberfläche (336) es an dem Ankerabschnitt (130) ist und
sich die sich verjüngende Oberfläche (336) in proximaler Richtung von der flachen Oberfläche (334) weg verengt.

7. Hämostatischer Gewebeanker nach einem der Ansprüche 1 bis 3, wobei die zwei oder mehr Scheiben (126, 326) ein Material umfassen, das konfiguriert ist, um ein therapeutisches Mittel zu eluieren, oder die zwei oder mehr Scheiben (126, 326) mit einem therapeutischen Mittel beschichtet sind.

8. Hämostatischer Gewebeanker nach einem der Ansprüche 1 bis 3, wobei die zwei oder mehr Scheiben (126) ein Hydrogel umfassen.

9. Hämostatischer Gewebeanker nach einem der Ansprüche 1 bis 3, wobei die zwei oder mehr Scheiben (126, 326) drei oder mehr Scheiben (126, 326) umfassen.

10. Hämostatischer Gewebeanker nach Anspruch 9, wobei die drei oder mehr Scheiben (126, 326) koaxial entlang der länglichen Ankerwelle (132) angeordnet sind.

11. Hämostatischer Gewebeanker nach einem der Ansprüche 1 bis 2, wobei die zwei oder mehr Scheiben (126, 326) verformbar sind, wenn der hämostatische Gewebeanker (120, 320, 420) innerhalb des Katheters zur Abgabe an die Zielstelle angeordnet ist.

12. Hämostatischer Gewebeanker nach einem der Ansprüche 1 bis 3, wobei die Herzgewebewand eine Myokardgewebewand ist und wobei die zwei oder mehr Scheiben (126, 326) konfiguriert sind, um vollständig innerhalb der Myokardgewebewand implantiert zu sein.

13. Ankersystem (150), umfassend den hämostatischen Gewebeanker (120, 320, 420) nach einem der Ansprüche 1 bis 3, wobei das Ankersystem (150) ferner einen am hämostatischen Gewebeanker (120 , 320, 420) befestigten Haltegurt (152) so umfasst, dass eine Zugkraft über den Haltegurt (152) auf den hämostatischen Gewebeanker (120, 320, 420) ausgeübt werden kann.

14. Ankersystem nach Anspruch 13, wobei der hämostatische Gewebeanker (120, 320, 420) ferner ein längliches Zugelement (146) umfasst, das mit einem Abschnitt des Ankerabschnitts (130) verbunden ist und wobei der Haltegurt (152) an dem längliche Zugelement (146) so befestigt ist, dass die Zugkraft über den Haltegurt (152) und das längliche Zugelement (146) auf den Ankerabschnitt (130) ausgeübt werden kann.

15. Ankersystem nach Anspruch 13, ferner umfassend einen zweiten Gewebeanker (133), der vom hämostatischen Gewebeanker (120, 320, 420) getrennt und verschieden ist, wobei der zweite Gewebeanker (133) durch den Haltegurt (152) mit dem hämostatischen Gewebeanker (120, 320, 420) verbunden werden kann oder mit diesem verbunden ist.

## Revendications

1. Ancrage tissulaire hémostatique (120, 320, 420) pouvant être administré à l'intérieur d'un cathéter à un site cible, l'ancrage tissulaire hémostatique (120, 320, 420) étant conçu pour être ancré à une paroi de tissu cardiaque au niveau du site cible, l'ancrage tissulaire hémostatique (120, 320, 420) comprenant :
une partie d'ancrage (130) supportée par une tige d'ancrage généralement allongée (132) pouvant être fixée de manière amovible au cathéter, la partie d'ancrage (130) étant positionnée au niveau d'une extrémité distale de la tige d'ancrage généralement allongée (132), la partie d'ancrage (130) étant conçue pour se déployer d'une première configuration généralement allongée à une seconde configuration déployée de sorte que la partie d'ancrage (130) dans la seconde configuration déployée puisse être étirée étroitement contre la paroi de tissu cardiaque au niveau du site cible lorsqu'une force de traction est appliquée à la partie d'ancrage (130) ; et
deux disques ou plus (126, 326) couplés à la tige d'ancrage allongée (132), les deux disques ou plus (126, 326) entourant la tige d'ancrage allongée (132), les deux disques ou plus (126, 326) étant conçus pour être disposés entièrement à l'intérieur de la paroi de tissu cardiaque au niveau du site cible,
moyennant quoi, une fois que les deux disques ou plus (126, 326) sont implantés entièrement à l'intérieur de la paroi de tissu cardiaque au niveau du site cible, les deux disques ou plus (126, 326) agissent comme un joint hémostatique d'une ouverture à travers la paroi de tissu cardiaque, ouverture à travers laquelle est disposée la tige d'ancrage allongée (132), **caractérisé en ce que** :
l'ancrage tissulaire hémostatique (120, 320, 420) comprend en outre un manchon (124, 330) entourant au moins une partie de la tige d'ancrage allongée (132), et lesdits deux disques ou plus (126, 326) faisant partie intégrante du manchon (124, 330) et étant couplés à la tige d'ancrage allongée (132) via le manchon (124, 330).

2. Ancrage tissulaire hémostatique selon la revendication 1, lesdits deux disques ou plus (126, 326) étant mous et flexibles.

3. Ancrage tissulaire hémostatique selon la revendication 1, lesdits deux disques ou plus (326) étant rigides.

4. Ancrage tissulaire hémostatique selon l'une quelconque des revendications 1 à 3, lesdits deux disques ou plus (126) comprenant un matériau poreux.

5. Ancrage tissulaire hémostatique selon l'une quelconque des revendications 1 à 3, lesdits deux disques ou plus (126, 326) comprenant un biopolymère.

6. Ancrage tissulaire hémostatique selon l'une quelconque des revendications 1 à 3 :
chacun desdits deux disques ou plus (326) étant profilé de manière à définir une surface plane (334) et une surface biseautée (336),
la surface plane (334) étant plus proche de la partie d'ancrage (130) que la surface biseautée (336) ne l'est de la partie d'ancrage (130), et
la surface biseautée (336) se rétrécissant dans une direction proximale s'éloignant de la surface plane (334).

7. Ancrage tissulaire hémostatique selon l'une quelconque des revendications 1 à 3, lesdits deux disques ou plus (126, 326) comprenant un matériau qui est conçu pour éluer un agent thérapeutique, ou lesdits deux disques ou plus (126, 326) étant enduits d'un agent thérapeutique.

8. Ancrage tissulaire hémostatique selon l'une quelconque des revendications 1 à 3, lesdits deux disques ou plus (126) comprenant un hydrogel.

9. Ancrage tissulaire hémostatique selon l'une quelconque des revendications 1 à 3, lesdits deux disques ou plus (126, 326) comprenant trois disques ou plus (126, 326).

10. Ancrage tissulaire hémostatique selon la revendication 9, lesdits trois disques ou plus (126, 326) étant disposés coaxialement le long de la tige d'ancrage allongée (132).

11. Ancrage tissulaire hémostatique selon l'une quelconque des revendications 1 à 2, lesdits deux disques ou plus (126, 326) étant déformables lorsque l'ancrage tissulaire hémostatique (120, 320, 420) est disposé à l'intérieur du cathéter pour l'administration au site cible.

12. Ancrage tissulaire hémostatique selon l'une quelconque des revendications 1 à 3, ladite paroi de tissu cardiaque étant une paroi de tissu myocardique, et lesdits deux disques ou plus (126, 326) étant conçus pour être implantés entièrement à l'intérieur de la paroi de tissu myocardique.

13. Système d'ancrage (150) comprenant l'ancrage tissulaire hémostatique (120, 320, 420) selon l'une quelconque des revendications 1 à 3, ledit système d'ancrage (150) comprenant en outre une attache (152) fixée à l'ancrage tissulaire hémostatique (120, 320, 420) de sorte qu'une force de traction puisse être appliquée à l'ancrage tissulaire hémostatique (120, 320, 420) via l'attache (152).

14. Système d'ancrage selon la revendication 13, ledit ancrage tissulaire hémostatique (120, 320, 420) comprenant en outre un élément de tension allongé (146) couplé à une partie de la partie d'ancrage (130) et ladite attache (152) étant fixée à l'élément de tension allongé (146) de sorte que la force de traction puisse être appliquée à la partie d'ancrage (130) via l'attache (152) et l'élément de tension allongé (146).

15. Système d'ancrage selon la revendication 13, comprenant en outre un second ancrage tissulaire (133) séparé et distinct de l'ancrage tissulaire hémostatique (120, 320, 420), ledit second ancrage tissulaire (133) pouvant être couplé ou étant couplé à l'ancrage tissulaire hémostatique (120, 320, 420) par l'attache (152).
